# EUROPÄISCHE PATENTSCHRIFT

(11) **EP 0 325 137 B1**
(45) Veröffentlichungstag und Bekanntmachung des Hinweises auf die Patenterteilung: **14.07.1993**
(21) Anmeldenummer: 89100294.1
(22) Anmeldetag: 10.01.1989
(51) Int. Cl.: C12P 1/02, C12P 17/02, C12P 17/18

(54) **Verfahren zur Herstellung von Farbstoffen und/oder Wirkstoffen in der wasserarmen, sterilen Wirbelschicht**
Process for preparing dyes and/or active substances in a sterile fluidised bed poor in water
Procédé de préparation de colorants et/ou substances actives dans un lit fluidisé stérile et appauvri en eau

(30) Priorität: 21.01.1988 DE 3801588
(43) Veröffentlichungstag der Anmeldung: 26.07.1989
(73) Patentinhaber: HOECHST AKTIENGESELLSCHAFT, 65926 Frankfurt am Main (DE)
(72) Erfinder: Bayer, Thomas, Dr., D-3000 Hannover (DE); Buchholz, Rainer, Dr., D-5239 Unnau (DE); Deger, Hans-Matthias, Dr., D-6238 Hofheim am Taunus (DE); Wink, Joachim, Dr., D-6050 Offenbach (DE)

(56) Entgegenhaltungen:
- EP-A- 0 106 146
- US-A- 4 046 921
- AGR. BIOL. CHEM., Band 39, Nr. 9, 1975, Seiten 1789-1795; M. YOSHIMURA et al.: "Production of Monascus-pigment in a submerged culture"
- CHEMICAL ABSTRACTS, Band 83, Nr. 1, 7. Juli 1975, Seite 621, Zusammenfassung Nr. 7183j, Columbus, Ohio, US

## Beschreibung

Gegenstand der Erfindung ist ein Verfahren zur Herstellung von Farbstoffen und/oder Wirkstoffen durch Kultivierung eines Mikroorganismus der Gattung Monascus in der wasserarmen, sterilen Wirbelschicht. Die Kultivierung der Mikroorganismen wird in einem sterilisierbaren Wirbelschichtfermenter durchgeführt, wie er in der Deutschen Patentanmeldung P 36 25 698.6 vorgeschlagen wurde.

Die vielfältigen Anwendungsmöglichkeiten von Reaktionen im Wirbelschichtfermenter zeigt die Europäische Patentanmeldung 106 146, in der eine Vielzahl unterschiedlicher mikrobieller Metabolite und Enzyme durch fermentative Umsetzung von Präkursoren oder durch Zusatz von Effektoren auf biologischem Wege hergestellt werden. Die Mikroorganismen werden dabei in ein wirbelfähiges Granulat überführt. Die Präkursoren oder Effektoren werden entweder vorher in das Granulat eingearbeitet oder als Lösung oder Suspension auf das Granulat im Wirbelschichtfermenter aufgesprüht. Mit einem derartigen Verfahren lassen sich Substanzen in annehmbaren Ausbeuten herstellen, die durch chemischsynthetische Methoden nur schwer oder gar nicht zugänglich sind.

Die fermentative Herstellung von pharmakologischen Wirkstoffen unter Verwendung von Mikroorganismen der Gattung Monascus ist beispielsweise in der Deutschen Offenlegungsschrift DE 30 06 215 dargelegt. Dort ist die Herstellung von Monacolin K, einer antihypercholesterinämischen Substanz, beschrieben.

Die Herstellung und Verwendung von Pigmenten, die durch Kultivierung von Mikroorganismen der Gattung Monascus erhalten wurden, ist Gegenstand von einer Reihe von Patenten (US 4,031,250; US 3,765,906; DE-B 27 24 992; DE-B 24 61 642; FR-B 2 505 856).

Seit geraumer Zeit werden in Südostasien Mikroorganismen der Gattung Monascus zur Erzeugung roter Farbstoffe für die Lebensmittelfärbung verwendet. Die Kultivierung erfolgt auf Reis, wobei das Fermentationsgut in gewissen Zeitabständen durchmischt wird.

Bedingt durch diese einfache Technik ist nur eine unzureichende Versorgung der Pilze mit Sauerstoff möglich. Auch die Gefahr der Kontamination durch Fremdkeime ist bei diesem insterilen Verfahren nicht auszuschließen.

Die Aufarbeitung der Biomasse kann extraktiv vorgenommen werden. Zahlreiche organische Lösungsmittel wie Methanol, Ethanol, Aceton oder Essigester sind zur Extraktion der in Wasser schwer löslichen Farbstoffe geeignet.

In Asien wird auch die gesamte getrocknete Biomasse als roter Lebensmittelzusatz verwendet.

Weiterhin sind Verfahren bekannt, um die Farbstoffe wasserlöslicher zu machen. Dazu werden die Pigmente mit den unterschiedlichsten stickstoffhaltigen organischen Verbindungen, wie z. B. Aminosäuren, Proteinen oder Nucleinsäuren umgesetzt.

In jüngerer Zeit wurden auch submerse Verfahren entwickelt, wie z. B. in der Deutschen Patentschrift DE 27 24 992 ausgeführt worden ist.
Nachteilig an submersen Verfahren ist die starke Verdünnung der gebildeten Produkte und die in vielen Fällen großen Wassermengen, die die Aufarbeitung aufwendig gestalten. Weiterhin ist der Einsatz eines den natürlichen Oberflächenverfahren nahekommenden Verfahrens wünschenswert. Diese Bedingung erfüllt das Wirbelschichtverfahren, welches darüber hinaus steril durchgeführt werden kann (Deutsche Patentanmeldung P 36 25 698.6) und damit eine Kontaminierungsgefahr durch andere Mikroorganismen oder deren eventuell toxischen Stoffwechselprodukte verhindert wird. Dies ist besonders für Pharmazeutika und den Einsatz von Farbstoffen im Lebensmittelbereich wichtig.

### Die Erfindung betrifft nun:

Ein Verfahren zur Herstellung von Farbstoffen und/oder Wirkstoffen, wobei ein Mikroorganismus der Gattung Monascus in der wasserarmen, sterilen Wirbelschicht kultiviert wird, so lange, bis sich der Farbstoff und/oder der Wirkstoff in der Kultur anhäuft und wobei ggfs. anschließend der Farbstoff und/oder der Wirkstoff isoliert wird.

Unter Wirkstoffen werden biologisch aktive Substanzen verstanden, insbesondere Pharmazeutika wie Monacolin K.

Als Mikroorganismen der Gattung Monascus können beispielsweise eingesetzt werden:
Monascus albidus, M. albus, M. anka, M. araneous, M. bisporus, M. kaoliang, M. major, M. paxii, M. pilosus, M. pubigerus, M. ruber, M. purpureus, M. rubiginosus, M. rubropunctatus, M. serorubescens, M. vitreus oder deren Mutanten oder Varianten. Insbesondere geeignet sind: M. purpureus CBS 10907, M. ruber CBS 34650 und M. ruber CBS 13560. Mutanten oder Varianten erzeugt man in an sich bekannter Weise durch physikalische Mittel, beispielsweise durch Bestrahlung mit Ultraviolett- oder Röntgenstrahlen oder durch chemische Mutagene wie Methansulfonsäureethylester (Ethylmethylsulfonat, EMS) oder 2-Hydroxy-4-methoxybenzophenon (MOB).

Zur Durchführung des Verfahrens eignet sich ein sterilisierbarer Wirbelschichtfermenter, wie er in der Deutschen Patentanmeldung P 36 25 698.6 vorgeschlagen wurde, auf die an dieser Stelle ausdrücklich Bezug genommen wird.

Vor Beginn der Fermentation wird der Fermenter z. B. durch Einleiten von Heißdampf sterilisiert. In den so vorbereiteten Wirbelschichtfermenter wird nun nicht etwa die fertige Biomasse des in einem anderen Fermenter angezüchteten Mikroorganismus eingefüllt, was mit Sicherheit zu einer Kontamination mit Fremdkeimen führen würde, sondern der Wirbelschichtfermenter wird nur mit einer in einem Kleinfermenter herangezüchteten submersen Impfkultur unter den üblicherweise angewendeten sterilen Bedingungen beschickt. Der Mikroorganismus kann beispielsweise nach zwei verschiedenen Verfahren vermehrt werden. Im Blasensäulenbetrieb submers in einem flüssigen Nährmedium, wobei Pellets angezüchtet werden, die ohne Träger als wirbelfähige Partikel vermehrt werden können. Im anderen Fall wird ein fester Träger, z. B. Reis, Hirse oder andere Getreidearten oder auch Sinterglas zum Aufwachsen der Mikroorganismen benutzt, wobei die Trägerstoffe gleichzeitig auch Substrat sein können. In diesem Fall befinden sich die Mikroorganismen auch in der Aufwachsphase in der Wirbelschicht.

Sobald sich im Blasensäulenbetrieb eine ausreichende Menge von Mikroorganismen gebildet hat, wird die verbrauchte Substratlösung über Filterelemente, die in der Nähe des Fermenterbodens installiert sind, entfernt. Mit dieser Verfahrensweise ist sichergestellt, daß der Wirbelschichtfermenter jetzt eine Kultur eines Mikroorganismus enthält, die nicht durch Fremdkeime verunreinigt ist. Zur Durchführung der eigentlichen Wirbelschichtreaktion wird die bis zum Erreichen einer breiigen Konsistenz von der verbrauchten Kulturbrühe befreiten Masse der Mikroorganismen durch Einleiten eines Gasstromes von unten aufgewirbelt. Dazu wird Druckluft oder Sauerstoff verwendet, welche zuvor durch Sterilfilter gepreßt wurden, so daß durch den Gasstrom keine fremden Mikroorganismen in den Wirbelschichtreaktor hineingetragen werden können.

Das zum Aufwirbeln des biologischen Materials verwendete Gas kann im Kreis gefahren werden. Der Gasstrom wird dann in einen thermostatisierbaren Luftbefeuchter geleitet und dann das abgekühlte und mit Feuchtigkeit gesättigte Gas erneut in den Fermenter eingeleitet. Alternativ kann der Gasstrom auch im Durchlauf geführt werden.

Die zum Wachstum während der Wirbelschichtfermentation benötigte Nährlösung befindet sich in einem Vorratsbehälter und wird in fein verteilter Tröpfchenform in den Reaktor eingesprüht, es sei denn, die Mikroorganismen benutzen den Nährstoffe enthaltenden Träger, auf dem sie aufgewachsen sind, als ausschließliche Nährstoffquelle.

Besondere Vorsorge muß dafür getroffen werden, daß die Mikroorganismen nicht austrocknen. Hierfür dient der thermostatisierbare Luftbefeuchter, durch den sichergestellt wird, daß der eingeleitete Gasstrom mit Feuchtigkeit gesättigt ist. Darüber hinaus kann in den Fermenter, falls erforderlich, jederzeit keimfreies Wasser zugeführt und damit die Mikroorganismen befeuchtet werden.

Während der Fermentation nimmt der CO₂-Gehalt des Kreisgases ständig zu und der Sauerstoffgehalt entsprechend ab. Eine konstante Zusammensetzung des Gases ist aber für eine optimale Fermentation Voraussetzung. Deshalb ist der Wirbelschichtfermenter mit Kontrollelementen ausgestattet, die die kontinuierliche Entfernung des Kohlendioxids regeln. Die ausgeschleusten Gasmengen werden durch Zuführung von Sauerstoff oder Luft über eine untere Öffnung ersetzt.

Aus der erhaltenen Zellmasse lassen sich - sofern erforderlich - die Pigmente und/oder Wirkstoffe leicht nach bekannten Verfahren isolieren, beispielsweise durch Extraktion mit einem geeigneten Lösungsmittel wie Hexan, Methanol, Ethanol, Aceton oder Essigester. Diese Extraktion kann auch im Fermenter erfolgen, mit oder ohne vorheriger Trocknung der Biomasse.

In den nachfolgenden Beispielen wird das erfindungsgemäße Verfahren detailliert beschrieben.

### Beispiel 1

Der Stamm Monascus purpureus CBS 10907 wird in einem Kleinfermenter mit 15 l Inhalt in 2 - 3 Tagen zu einer Impfkultur herangezüchtet. Das Medium enthält 10 g/l Reis. Die Anzucht erfolgt bei 30°C, bei Luftbegasung (7,5 l/min) und einer Rührerdrehzahl von 500 UpM.

Diese Vorkultur wird zur Beimpfung des Wirbelschichtfermenters gemäß Deutscher Patentanmeldung P 36 25 698.6 benutzt, der zuvor bei 121°C für 30 min, mit 25 kg Reis befüllt und befeuchtet, mit Dampf sterilisiert wurde.

Bei einer Betriebstemperatur von 30°C wird nach einer Kultivierungsdauer von 7 Tagen 60 kg Feuchtmasse erhalten. Die Pigmente lassen sich daraus mit Aceton extrahieren.

### Beispiel 2

Der Stamm Monascus ruber CBS 34650 wird im Wirbelschichtreaktor auf einem synthetischen Medium so angezüchtet, daß Pellets entstehen. Nach dem Verbrauch der Nährstoffe wird die Substratlösung über Filterelemente abgelassen und die Pellets werden aufgewirbelt. Die Nährstoffversorgung im Wirbelbett erfolgt durch Aufsprühen einer sterilen Nährlösung. Die Fermentation wird nach 6 Tagen beendet. Die Pigmente werden extraktiv mit Aceton von der Biomasse abgetrennt.

## Patentansprüche

1. Verfahren zur Herstellung von Farbstoffen (Pigmenten) und/oder Wirkstoffen, dadurch gekennzeichnet, daß ein Mikroorganismus der Gattung Monascus in der wasserarmen, sterilen Wirbelschicht kultiviert wird, so lange, bis sich der Farbstoff (das Pigment) und/oder der Wirkstoff in der Kultur anhäuft und daß gegebenenfalls anschließend der Farbstoff (das Pigment) und/oder der Wirkstoff isoliert wird.

2. Verfahren nach Anspruch 1, dadurch gekennzeichnet, daß ein Mikroorganismus, ausgewählt aus:
Monascus albidus, M. albus, M. anka, M. araneous, M. bisporus, M. kaoliang, M. major, M. paxii, M. pilosus, M. pubigerus, M. ruber, M. purpureus, M. rubiginosus, M. rubropunctatus, M. serorubescens, M. vitreus oder deren Mutanten oder Varianten eingesetzt wird.

3. Verfahren nach Anspruch 1 oder 2, dadurch gekennzeichnet, daß die Mikroorganismen M. purpureus CBS 10907, M. ruber CBS 34650 oder M. ruber CBS 13560 auf festen Trägern verwendet werden.

4. Verfahren nach einem oder mehreren der Ansprüche 1 - 3, dadurch gekennzeichnet, daß die Mikroorganismen ohne feste Träger in Form von Pellets kultiviert werden.

5. Verfahren nach einem oder mehreren der Ansprüche 1 - 5, dadurch gekennzeichnet, daß die Extraktion der Farbstoffe und/oder Wirkstoffe im Wirbelschichtreaktor erfolgt.

## Claims

1. A process for the preparation of colorants (pigments) and/or active compounds, wherein a microorganism of the genus Monascus is cultures in a sterile fluidized bed of low water content until the colorant (the pigment) and/or the active compound accumulates in the culture and, if appropriate, the colorant (the pigment) and/or the active compound is then isolated.

2. The process as claimed in claim 1, wherein a microorganism selected from the group comprising: Monascus albidus, M. albus, M. anka, M. araneous, M. bisporus, M. kaoliang, M. major, M. paxii, M. pilosus, M. pubigerus, M. ruber, M. purpureus, M. rubiginosus, M. rubropunctatus, M. serorubescens, M. vitreus or mutants or variants thereof is used.

3. The process as claimed in claim 1 or 2, wherein the microorganisms M. purpureus CBS 10907, M. ruber CBS 34650 or M. ruber CBS 13560 on solid carriers are used.

4. The process as claimed in one or more of claims 1 to 3, wherein the microorganisms are cultured in the form of pellets without solid carriers.

5. The process as claimed in one or more of claims 1 to 4, wherein the extraction of the colorants and/or active compounds takes place in the fluidized-bed reactor.

## Revendications

1. Procédé de préparation de colorants (pigments) et/ou de substances actives, caractérisé en ce qu'on effectue la culture d'un microorganisme du genre Monascus dans un lit fluidisé stérile appauvri en eau, aussi longtemps qu'il le faut pour que le colorant (le pigment) et/ou la substance active s'accumule(nt) dans la culture, et qu'on isole éventuellement ensuite le colorant (le pigment) et/ou la substance active.

2. Procédé selon la revendication 1, caractérisé en ce qu'on utilise un microorganisme choisi parmi: Monascus albidus, M. albus, M. anka, M. aranecosus, M. bisporus, M. kaoliang. M. major, M. paxii, M. pilosus, M. pubigerus, M. ruber, M. purpureus, M. rubiginosus, M. rubropunctatus, M. serorubescens, M. vitreus, ou leurs mutants ou variantes.

3. Procédé selon la revendication 1 ou 2,, caractérisé en ce qu'on utilise les microorganismes M. purpureus CBS 10907, M. ruber CBS 34650 et M. ruber CBS 13560 sur des supports solides.

4. Procédé selon une ou plusieurs des revendications 1 à 3, caractérisé en ce que les microorganismes sont cultivés sous forme de boulettes, sans supports solides.

5. Procédé selon une ou plusieurs des revendications 1 à 4, caractérisé en ce que l'extraction des colorants et/ou des substances actives se déroule dans le réacteur à lit fluidisé.
